# EUROPEAN PATENT APPLICATION

(11) **EP 4 631 930 A1**
(43) Date of publication of application: **15.10.2025**
(21) Application number: 23912335.9
(22) Date of filing: 28.12.2023
(51) Int. Cl.: C07C 281/02

(54) **N-FLUOROALKYL COMPOUND PRODUCTION METHOD**

(30) Priority: 28.12.2022 JP 2022212369
(71) Applicant: Daikin Industries, Ltd., Osaka-shi, Osaka 530-0001 (JP); Japan Tobacco Inc., Tokyo 105-6927 (JP)
(72) Inventor: SHIRAI, Atsushi, Osaka-Shi, Osaka 530-0001 (JP); KUROKI, Yoshichika, Osaka-Shi, Osaka 530-0001 (JP); KISHIKAWA, Yosuke, Osaka-Shi, Osaka 530-0001 (JP); MANABE, Tomoyuki, Takatsuki-shi Osaka 569-1125 (JP)
(74) Representative: Hoffmann Eitle
(86) International application number: PCT/JP2023/047354
(87) International publication number: WO 2024/143558

(57) **Abstract**

Provided is a production method comprising step A of reacting a compound represented by Formula (2): 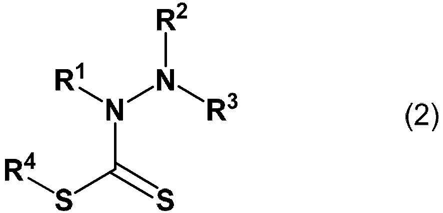 wherein R¹, R², and R³ are the same or different, R¹ represents -COR, R² and R³ each represent a hydrogen atom or -COR, and R⁴ represents a C₁-C₁₂ alkyl group or a C₆-C₁₂ aryl group; with a fluorinating agent containing IF₅, an amine, and HF at 80°C or lower throughout the reaction from the start to the end. This production method is capable of synthesizing a compound (N-fluoroalkylated hydrazine derivative) represented by formula (1): 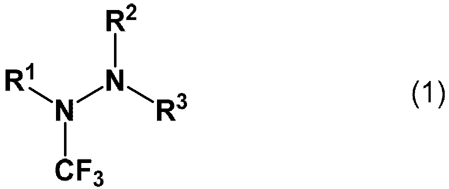 wherein R¹, R², and R³ are as defined above, wherein Rs are the same or different and each represents a C₁₋C₁₂ alkoxy group optionally substituted with one or more substituents or a C₆-C₁₂ aryloxy group optionally substituted with one or more substituents.

## Description

### Technical Field

The present disclosure relates a method for producing an N-fluoroalkyl compound.

### Background Art

N-fluoroalkylated hydrazine derivatives typified by, for example, a compound represented by the following formula: 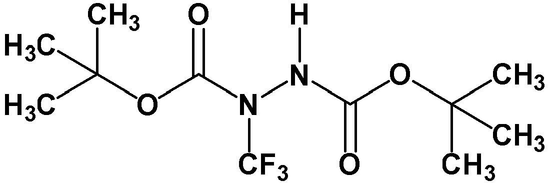 are useful as pharmaceutical intermediates etc.

For example, NPL 1 discloses a method for synthesizing an N-fluoroalkylated hydrazine derivative by the reaction below using di-tert-butyl azodicarboxylate as a substrate.

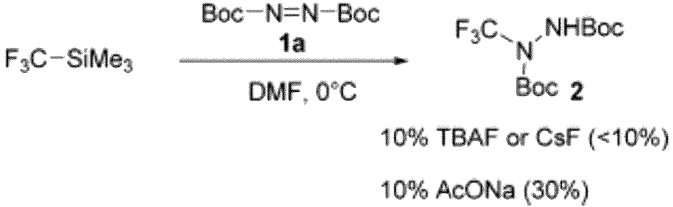

### Citation List

### Non-patent Literature

NPL 1: J. Org. Chem., 2015, 80, 1964-1971.

### Summary of Invention

### Technical Problem

An object of the present disclosure is to provide a method for efficiently synthesizing an N-fluoroalkylated hydrazine derivative.

### Solution to Problem

The present disclosure includes the following embodiments.

### Item 1

A method for producing a compound represented by formula (1): 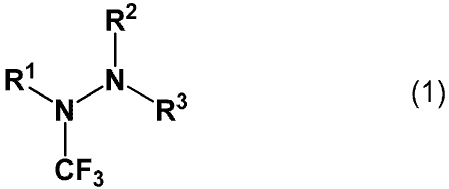 wherein
R¹, R², and R³ are the same or different,
R¹ represents -COR, and
R² and R³ each represent a hydrogen atom or -COR,
wherein Rs are the same or different and each represents a C₁₋C₁₂ alkoxy group optionally substituted with one or more substituents or a C₆-C₁₂ aryloxy group optionally substituted with one or more substituents;
the method comprising step A of reacting a compound represented by formula (2): 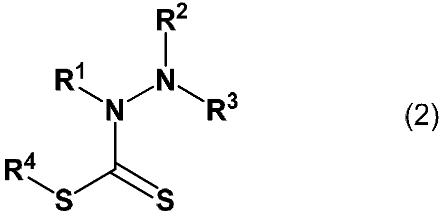 wherein
R¹, R², and R³ are as defined above, and
R⁴ represents a C₁₋C₁₂ alkyl group or a C₆-C₁₂ aryl group,
with a fluorinating agent containing IF₅, an amine, and HF at 80°C or lower throughout the reaction from the start to the end.

### Item 2

The production method according to Item 1, wherein in formulas (1) and (2),
R¹ represents a tert-butyloxycarbonyl group, a benzyloxycarbonyl group, a (2,2,2-trichloroethoxy)carbonyl group, an ethoxycarbonyl group, or an isopropoxycarbonyl group, and
R² and R³ are the same or different and each represents a hydrogen atom, a tert-butyloxycarbonyl group, a benzyloxycarbonyl group, a (2,2,2-trichloroethoxy)carbonyl group, an ethoxycarbonyl group, or an isopropoxycarbonyl group.

### Item 3

The production method according to Item 1 or 2, wherein the compound represented by formula (2) is

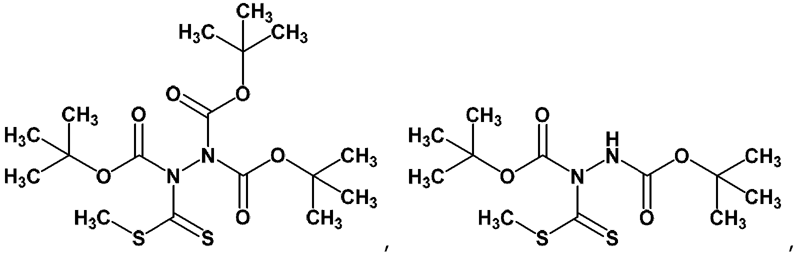

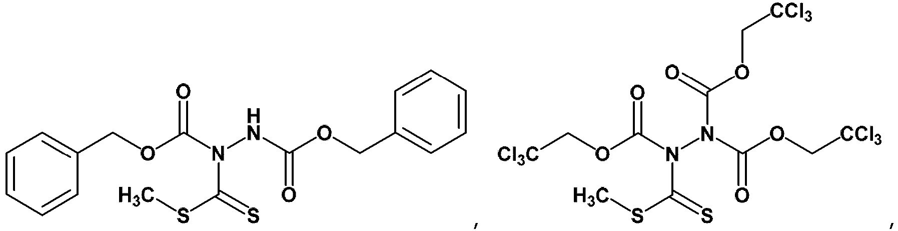

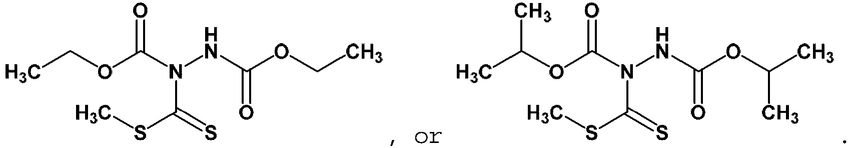

### Item 4

The production method according to any one of Items 1 to 3, wherein the compound represented by formula (2) is

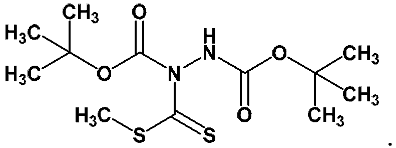

### Item 5

The production method according to any one of Items 1 to 4, wherein the compound represented by formula (1) is

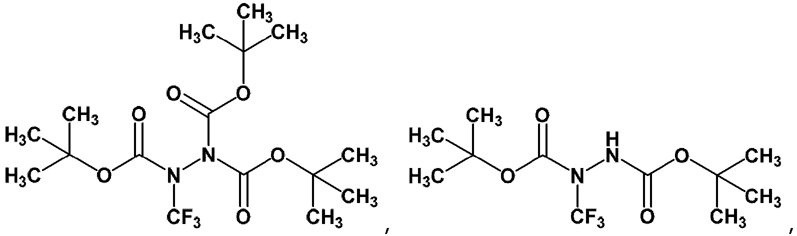

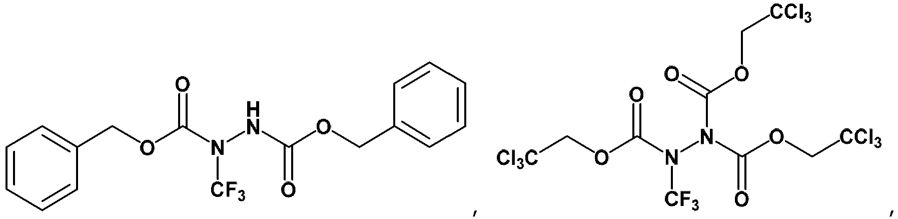

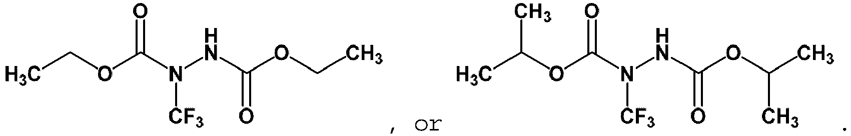

### Item 6

The production method according to any one of Items 1 to 5, wherein the compound represented by formula (1) is

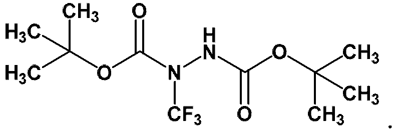

### Item 7

The production method according to any one of Items 1 to 6, wherein step A is carried out at a reaction temperature of 10 to 40°C.

### Item 8

The production method according to any one of Items 1 to 7, wherein step A is carried out in the presence of a solvent.

### Item 9

The production method according to Item 8, wherein the solvent is at least one member selected from the group consisting of (cyclo)alkanes, chlorine-containing organic solvents, aromatic solvents, nitrile-containing organic solvents, fluorine-containing organic solvents, and ester-containing organic solvents.

### Advantageous Effects of Invention

According to the present disclosure, an N-fluoroalkylated hydrazine derivative can be efficiently synthesized.

### Description of Embodiments

In the present specification, the terms "comprise," "contain," and "include" encompass the concepts of comprising, consisting essentially of, and consisting of.

In the present specification, a numerical range indicated by "A to B" means A or more, and B or less.

In the present disclosure, "yield" refers to the ratio (mol%) of the total molar amount of the target compound contained in the gas flowing out of the outlet of a reactor to the molar amount of the raw material compound supplied to the reactor.

The production method of the present disclosure is a method for producing a compound represented by formula (1): 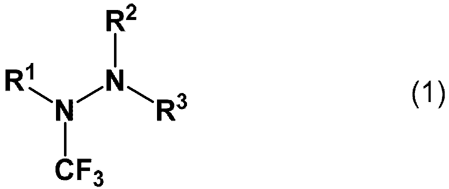 wherein
R¹, R², and R³ are the same or different,
R¹ represents -COR, and
R² and R³ each represent a hydrogen atom or -COR,
wherein Rs are the same or different and each represents a C₁₋C₁₂ alkoxy group optionally substituted with one or more substituents or a C₆-C₁₂ aryloxy group optionally substituted with one or more substituents;
the method comprising step A of reacting a compound represented by formula (2): 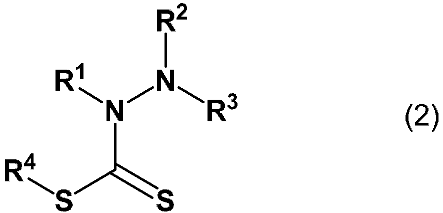 wherein
R¹, R², and R³ are as defined above, and
R⁴ represents a C₁-C₁₂ alkyl group or a C₆-C₁₂ aryl group,
with a fluorinating agent containing IF₅, an amine, and HF.

Conventional methods, such as the method of NPL 1, require the use of expensive raw materials and reagents, i.e., silane compounds and azo compounds; however, the present disclosure makes it possible to synthesize an N-fluoroalkylated hydrazine derivative, without using expensive raw materials or reagents.

### 1. Raw Material Compound

The raw material compound for use in the present disclosure is a compound represented by formula (2): 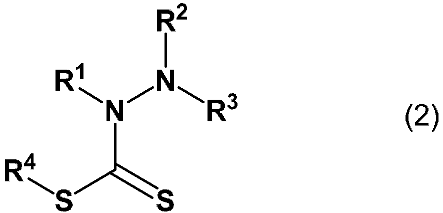 wherein
R¹, R², and R³ are the same or different,
R¹ represents -COR,
R² and R³ each represent a hydrogen atom or -COR,
wherein Rs are the same or different and each represents a C₁₋C₁₂ alkoxy group optionally substituted with one or more substituents or a C₆-C₁₂ aryloxy group optionally substituted with one or more substituents, and
R⁴ represents a C₁-C₁₂ alkyl group or a C₆-C₁₂ aryl group.

In formula (2), examples of the alkoxy group represented by R in -COR represented by R¹, R², and R³ include linear or branched C₁-C₁₂ (particularly C₁-C₈) alkoxy groups, such as a methoxy group, an ethoxy group, an n-propoxy group, an isopropoxy group, an n-butyloxy group, an isobutyloxy group, a sec-butyloxy group, a tert-butyloxy group, an n-pentyloxy group, a neopentyloxy group, an n-hexyloxy group, and a benzyloxy group.

The alkoxy group is optionally substituted. Examples of substituents for the alkoxy group include halogen atoms (e.g., a fluorine atom, a chlorine atom, a bromine atom, and an iodine atom) and the aryl groups mentioned below. When the alkoxy group has substituents, the number of the substituents is not limited and can be 1 to 6, and particularly 1 to 3.

In formula (2), examples of the aryloxy group represented by R in -COR represented by R¹, R², and R³ include monovalent groups in which an aryl group is bonded to an oxygen atom. Examples of aryl groups include those having 6 to 12 carbon atoms, such as a phenyl group, an o-tolyl group (2-methylphenyl group), an m-tolyl group (3-methylphenyl group), a p-tolyl group (4-methylphenyl group), a 1-naphthyl group, and a 2-naphthyl group. Specific examples of the aryloxy group include C₆-C₁₂ (particularly C₆-C₁₀) aryloxy groups, such as a phenoxy group, an o-tolyloxy group (2-methylphenyloxy group), an m-tolyloxy group (3-methylphenyloxy group), a p-tolyloxy group (4-methylphenyloxy group), a 1-naphthyloxy group, and a 2-naphthyloxy group.

The aryloxy group is optionally substituted. Examples of substituents for the aryloxy group include halogen atoms (e.g., a fluorine atom, a chlorine atom, a bromine atom, and an iodine atom), the alkoxy groups mentioned above, the aryl groups mentioned above, and the alkyl groups mentioned below. When the aryloxy group has substituents, the number of the substituents is not limited and can be 1 to 6, and particularly 1 to 3.

Among these, in formula (2), R¹ is preferably a protecting group for an amino group from the viewpoint of easily stabilizing the compound represented by formula (1) to be obtained and easily improving the yield of the compound represented by formula (1) to be obtained. Examples include a methoxycarbonyl group, an ethoxycarbonyl group, an isopropoxycarbonyl group, a tert-butyloxycarbonyl group (Boc), a benzyloxycarbonyl group (Cbz), a p-methoxybenzyloxycarbonyl group, a (2,2,2-trichloroethoxy)carbonyl group (Troc), and a p-methoxyphenyloxycarbonyl group. Among these, an ethoxycarbonyl group, an isopropoxycarbonyl group, a tert-butyloxycarbonyl group (Boc), a benzyloxycarbonyl group (Cbz), a (2,2,2-trichloroethoxy)carbonyl group (Troc), and the like are preferred, a tert-butyloxycarbonyl group (Boc), a benzyloxycarbonyl group (Cbz) and the like are more preferred, and a tert-butyloxycarbonyl group (Boc) is even more preferred.

In formula (2), R² and R³ may be a hydrogen atom or -COR. From the viewpoint of the yield etc. of the compound represented by formula (1) to be obtained, it is preferable that at least one of R² and R³ is -COR (particularly, a protecting group for an amino group), and it is more preferable that one of R² and R³ is a hydrogen atom and the other is -COR (particularly, a protecting group for an amino group). The protecting group for an amino group for use may be those mentioned above. That is, it is preferable that one of R² and R³ is a hydrogen atom and the other is an ethoxycarbonyl group, an isopropoxycarbonyl group, a tert-butyloxycarbonyl group (Boc), a benzyloxycarbonyl group (Cbz), a (2,2,2-trichloroethoxy)carbonyl group (Troc), or the like. It is more preferable that one of R² and R³ is a hydrogen atom and the other is a tert-butyloxycarbonyl group (Boc), a benzyloxycarbonyl group (Cbz), or the like. It is even more preferable that one of R² and R³ is a hydrogen atom and the other is a tert-butyloxycarbonyl group (Boc).

In formula (2), examples of the alkyl group represented by R⁴ include linear or branched C₁-C₁₂ (particularly C₁-C₈) alkyl groups, such as a methyl group, an ethyl group, an n-propyl group, an isopropyl group, an n-butyl group, an isobutyl group, a sec-butyl group, a tert-butyl group, an n-pentyl group, a neopentyl group, and an n-hexyl group.

The alkyl group is optionally substituted. Examples of substituents for the alkyl group include halogen atoms (e.g., a fluorine atom, a chlorine atom, a bromine atom, and an iodine atom), the alkoxy groups mentioned above, the aryl groups mentioned above, and the aryloxy groups mentioned above. When the alkyl group has substituents, the number of the substituents is not limited and can be 1 to 6, and particularly 1 to 3.

In formula (2), examples of the aryl group represented by R⁴ include C₆-C₁₂ (particularly C₆-C₁₀) aryl groups, such as a phenyl group, an o-tolyl group (2-methylphenyl group), an m-tolyl group (3-methylphenyl group), a p-tolyl group (4-methylphenyl group), a 1-naphthyl group, and a 2-naphthyl group.

The aryl group is optionally substituted. Examples of substituents for the alkyl group include halogen atoms (e.g., a fluorine atom, a chlorine atom, a bromine atom, and an iodine atom), the alkoxy groups mentioned above, and the aryl groups mentioned above. When the alkyl group has substituents, the number of the substituents is not limited and can be 1 to 6, and particularly 1 to 3.

In formula (2), R⁴ is preferably an alkyl group from the viewpoint of easily stabilizing the compound represented by formula (1) to be obtained and easily improving the yield of the compound represented by formula (1) to be obtained.

Examples of the raw material compounds that satisfy the above conditions include the following compounds: 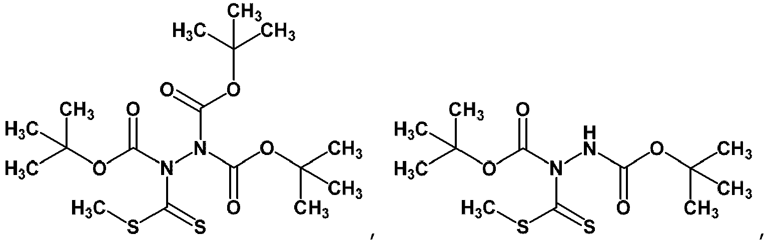 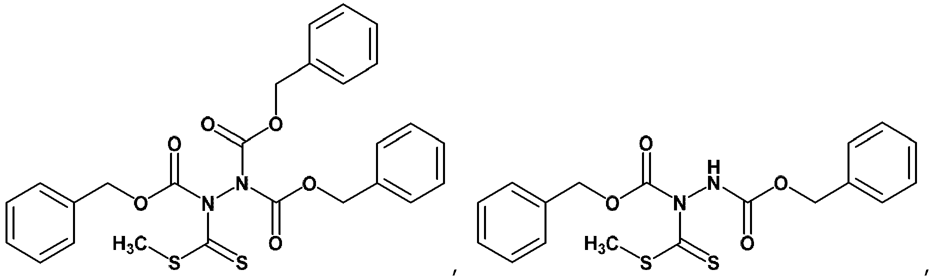 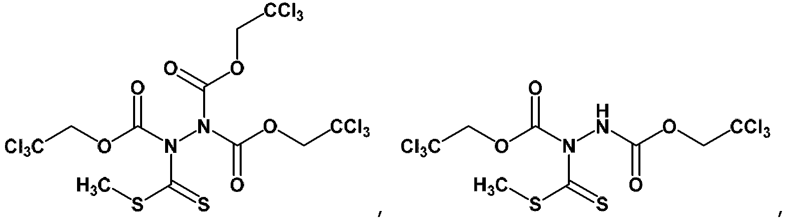 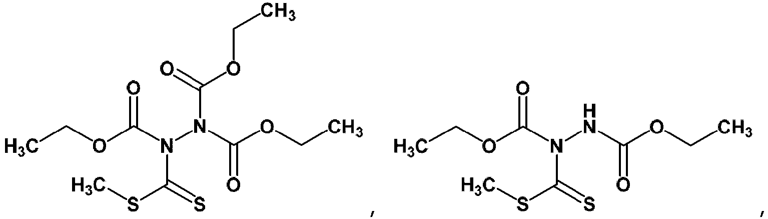 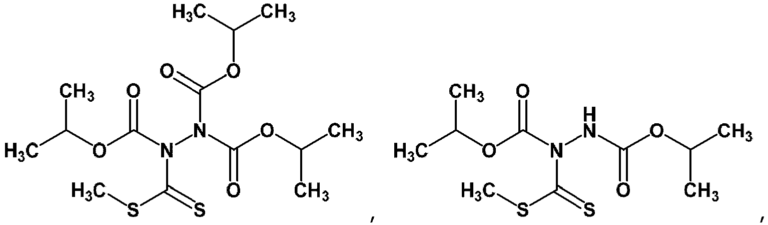 and the like. Among these, preferred are those shown in the Examples below, such as the following compounds: 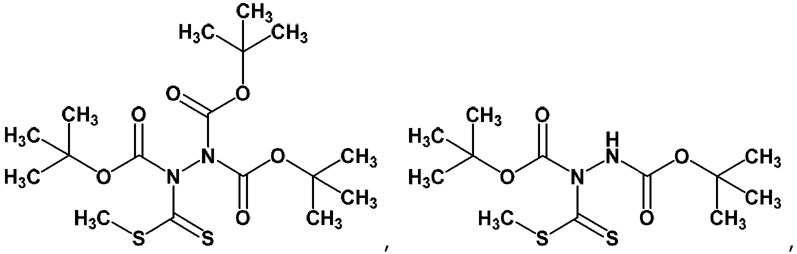 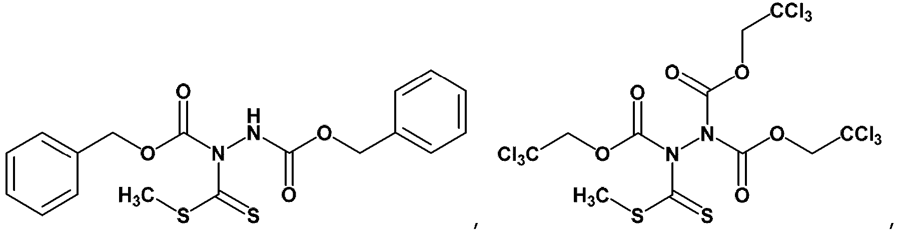 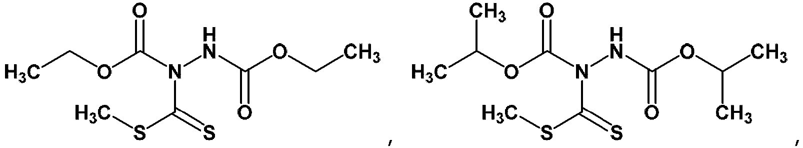 and the like, and more preferred is the following compound:

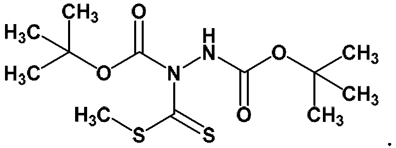

### 2. Fluorination Reaction

In step A of the production method of the present disclosure, a fluorination reaction of a compound represented by formula (2), which is the raw material compound, is carried out to fluorinate the dithiocarboxylate ester of the compound represented by formula (2) to form a trifluoromethyl group to thereby obtain a compound represented by formula (1).

### 3. Fluorinating Agent

The fluorinating agent for use in the present disclosure is a fluorinating agent containing IF₅, an amine, and HF (hydrogen fluoride).

IF₅ is a non-explosive, easy-to-handle liquid with a boiling point of 100.5°C and a melting point of 9.4°C, and is a fluorinating agent that can be used industrially. However, it is difficult to control the high oxidizing properties of IF₅ in fluorination. Although IF₅ has been used, for example, for the addition of IF to perfluoroolefins and for the substitution of iodine with fluorine in iodinated perfluoroolefins, there are not many reports on the fluorination reaction of organic compounds having hydroxyl groups, carbonyl groups, etc.

According to the present disclosure, a fluorinating agent containing IF₅, an amine, and HF (hydrogen fluoride) is used, whereby a fluorination reaction of a compound represented by formula (2), which is the raw material compound, is carried out to fluorinate the dithiocarboxylate ester of the compound represented by formula (2) to form a trifluoromethyl group to thereby obtain a compound represented by formula (1).

HF (hydrogen fluoride) used here as an acid may be supported on a variety of carriers. Examples of the carrier include SiO₂, methylated SiO₂, Al₂O₃, Al₂O₃-WB, MoO₃, ThO₂, ZrO₂, TiO₂, Cr₂O₃, SiO₂-Al₂O₃, SiO₂-TiO₂, SiO₂-ZrO₂, TiO₂-ZrO₂, Al₂O₃-B₂O₃, SiO₂-WO₃, SiO₂-NO₄F, HSO₃Cl-Al₂O₃, HF-NH₄-Y, HF-Al₂O₃, NH₄F-SiO₂-Al₂O₃, AlF₃-Al₂O₃, Ru-F-Al₂O₃, F-Al₂O₃, KF-Al₂O₃, AlPO₄, AlF₃, bauxite, kaolin, activated carbon, graphite, Pt-graphite, ion exchange resins, metal sulfates, chlorides, metals, such as Al, alloys, such as Al-Mg and Ni-Mo, and polymers, such as polystyrene.

The amount of HF (hydrogen fluoride) used as an acid in the present disclosure can be selected within the range from a catalytic amount to a large excess. From the viewpoint of easily stabilizing the compound represented by formula (1) to be obtained and easily improving the yield of the compound represented by formula (1) to be obtained, the amount is preferably 1 to 10 mol, and more preferably 3 to 6 mol, per mol of IF₅. In terms of HF (hydrogen fluoride) used as an acid, a hydrogen fluoride acid can also be used as a reaction solvent. In this case, the amount of the solvent may be a very small amount or a large excess.

Examples of amines used as a base in the present disclosure include aliphatic amines (aliphatic primary amines, aliphatic secondary amines, aliphatic tertiary amines), alicyclic amines (alicyclic secondary amines, alicyclic tertiary amines), aromatic amines, heterocyclic amine, and polymer-supported amines.

Examples of aliphatic primary amines include methylamine, ethylamine, propylamine, butylamine, pentylamine, hexylamine, cyclohexylamine, and ethylenediamine.

Examples of aliphatic secondary amines include dimethylamine, diethylamine, dipropylamine, dibutylamine, dipentylamine, dihexylamine, and dicyclohexylamine.

Examples of aliphatic tertiary amines include trimethylamine, triethylamine, diisopropylethylamine, and N,N,N',N'-tetramethylethylenediamine.

Examples of alicyclic secondary amines include piperidine, piperazine, pyrrolidine, and morpholine. Examples of alicyclic tertiary amines include N-methylpiperazine, N-methylpyrrolidine, 5-diazabicyclo[4.3.0]non-5-ene, and 1,4-diazabicyclo[2.2.2]octane.

Examples of aromatic amines include aniline, 2-methylaniline, 3-methylaniline, 4-methylaniline, N-methylaniline, 2,3-dimethylaniline, 2,6-dimethylaniline, 3,4-dimethylaniline, N,N-dimethylaniline, haloaniline, and nitroaniline.

Examples of heterocyclic amines include pyridine, pyrimidine, piperazine, quinoline, and imidazole.

Examples of polymer-supported amines include polyallylamine and polyvinylpyridine.

These amines can be used alone or in a combination of two or more.

The amount of the amine used as a base in the present disclosure can be selected within the range from a catalytic amount to a large excess. From the viewpoint of easily stabilizing the compound represented by formula (1) to be obtained and easily improving the yield of the compound represented by formula (1) to be obtained, the amount is preferably 0.1 to 5 mol, and more preferably 0.1 to 3 mol, per mol of IF₅.

In the present disclosure, when hydrogen fluoride is used as a reaction solvent and an amine is used as a base, a reaction may occur and one or more amine salts of hydrogen fluoride may be produced, such as ammonium fluoride, tetraethylammonium fluoride, tetrabutylammonium fluoride, and polyallylammonium fluoride. The present disclosure also encompasses embodiments in which such salts are formed.

In the present disclosure, the amount of the fluorinating agent used can be selected within the range from a catalytic amount to a large excess. From the viewpoint of easily stabilizing the compound represented by formula (1) to be obtained and easily improving the yield of the compound represented by formula (1) to be obtained, the amount is preferably 0.1 to 20 parts by mass, and more preferably 0.5 to 5 parts by mass, per 100 parts by mass of the compound represented by formula (2).

### 4. Solvent

The production method of the present disclosure is usually preferably carried out in the presence of a solvent from the viewpoint of reaction efficiency etc. As described above, it is possible to use HF (hydrogen fluoride) as a reaction solvent, and it is also possible to use a reaction solvent separately from hydrogen fluoride.

Examples of usable reaction solvents include, but are not limited to, (cyclo)alkanes, such as n-pentane, n-hexane, n-heptane, n-octane, cyclopentane, cyclohexane, cycloheptane, and cyclooctane; chlorine-containing organic solvents, such as dichloromethane and chloroform; aromatic solvents, such as benzene, toluene, and xylene; nitrile-containing organic solvents, such as acetonitrile, propionitrile, and benzonitrile; fluorine-containing organic solvents, such as perfluorohexane, perfluorooctane, perfluorodimethylcyclohexane, perfluorobenzene, and perfluorotoluene; and ester-containing organic solvents, such as ethyl acetate, propyl acetate, butyl acetate, methyl propionate, ethyl propionate, and butyl propionate. These organic solvents may be used alone or in a combination of two or more. Among these, from the viewpoint of the yield etc. of the compound represented by formula (1) to be obtained, (cyclo)alkanes, chlorine-containing organic solvents, aromatic solvents, nitrile-containing organic solvents, ester-containing organic solvents, and the like are preferred, alkanes, chlorine-containing organic solvents, aromatic solvents, nitrile-containing organic solvents, and the like are more preferred, and chlorine-containing organic solvents, aromatic solvents, and the like are even more preferred.

In the present disclosure, the amount of the solvent used can usually be an excess amount. From the viewpoint of easily stabilizing the compound represented by formula (1) to be obtained and easily improving the yield of the compound represented by formula (1) to be obtained, the amount of the solvent used is preferably 0.1 to 100 parts by mass, more preferably 0.1 to 10 parts by mass, and even more preferably 0.1 to 5 parts by mass, per part by mass of the compound represented by formula (2).

### 5. Other Conditions

In step A in the present disclosure, the reaction atmosphere when the raw material compound (the compound represented by formula (2)) is reacted with the fluorinating agent to obtain the compound represented by formula (1) is not limited, and can be, for example, an inert gas atmosphere, such as a nitrogen gas atmosphere or an argon gas atmosphere.

In step A in the present disclosure, the reaction temperature when the raw material compound (the compound represented by formula (2)) is reacted with the fluorinating agent to obtain the compound represented by formula (1) is 80°C or lower, preferably 70°C or lower, more preferably 60°C or lower, even more preferably 50°C or lower, and particularly preferably 40°C or lower throughout the reaction from the start to the end, from the viewpoint of easily stabilizing the compound represented by formula (1) to be obtained and easily improving the yield of the compound represented by formula (1) to be obtained. For the same reason, the reaction temperature can be usually -20°C or higher, preferably -10°C or higher, more preferably 0°C or higher, and further preferably 10°C or higher throughout the reaction from the start to the end. Therefore, the reaction temperature can be usually -20 to 80°C, preferably -10 to 70°C, more preferably 0 to 60°C, even more preferably 10 to 50°C, and particularly preferably 10 to 40°C throughout the reaction from the start to the end.

Specifically, it is preferable to maintain the reaction temperature within the above ranges from the start to the end of the reaction (from the time when the raw material compound (the compound represented by formula (2)) is brought into contact with the fluorinating agent to the time when the reaction is ended). In the present disclosure, the reaction between the raw material compound (the compound represented by formula (2)) and the fluorinating agent is an exothermic reaction. Thus, if the reaction is allowed to proceed at room temperature, the final reaction temperature would not be within the range of 80°C or lower, resulting in a low yield of the target compound.

In step A in the present disclosure, the reaction time when the raw material compound (the compound represented by formula (2)) is reacted with the fluorinating agent to obtain the compound represented by formula (1) can be set to allow for the reaction to sufficiently proceed and can be appropriately adjusted.

The compound represented by formula (1) is obtained as described above, and various purification treatments can be carried out as necessary according to conventional methods.

### 6. Target Compound

The target compound obtained as described above is a compound represented by formula (1): 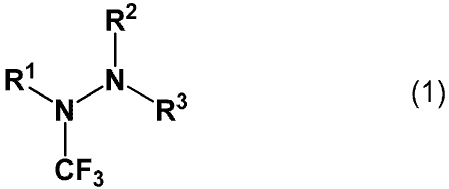 wherein
R¹, R², and R³ are the same or different,
R¹ represents -COR, and
R² and R³ each represent a hydrogen atom or -COR,
wherein Rs are the same or different and each represents a C₁₋C₁₂ alkoxy group optionally substituted with one or more substituents or a C₆-C₁₂ aryloxy group optionally substituted with one or more substituents.

In formula (1), R¹, R², and R³ may be those described above. Preferable specific examples are also the same.

Therefore, examples of the target compound obtained according to the production method of the present disclosure include the following compounds: 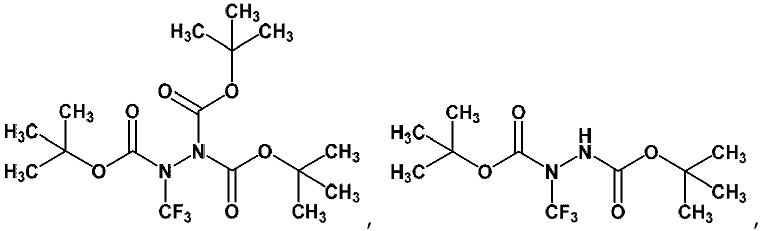 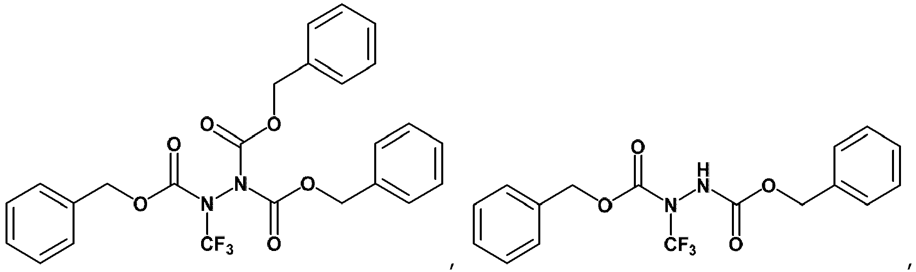 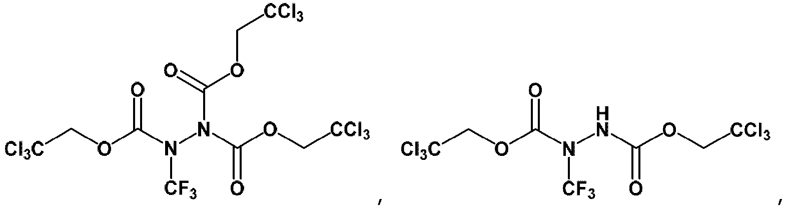 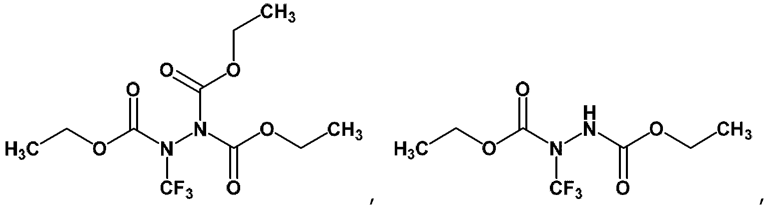 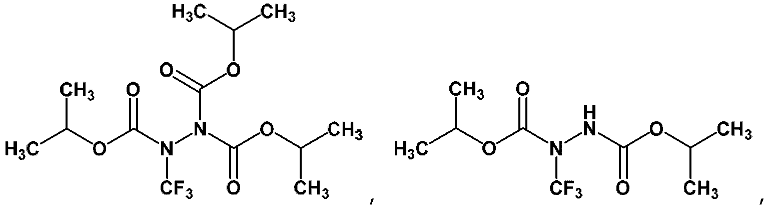 and the like. Among these, preferred are those shown in the Examples below, such as the following compounds: 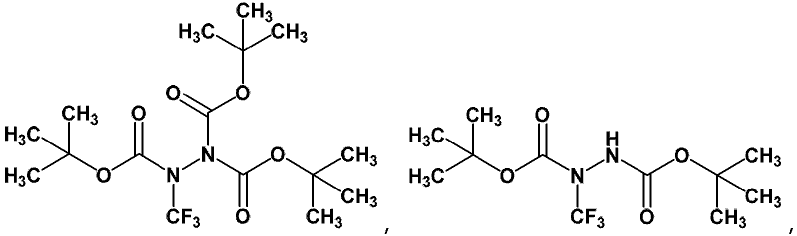 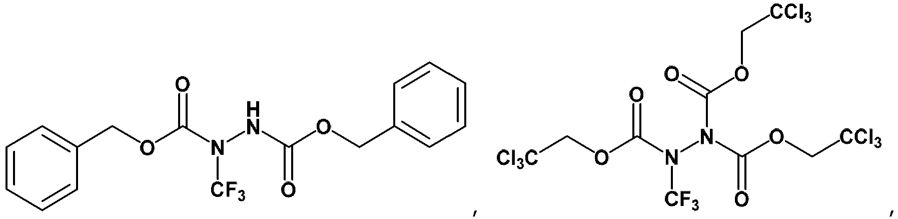 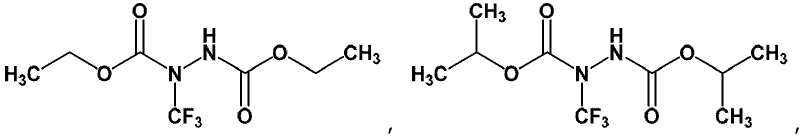 and the like, and more preferred is the following compound:

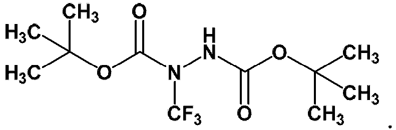

In particular, when the compound represented by formula (1) is the following compound: 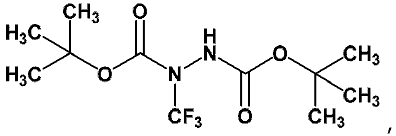 and the compound represented by formula (2) is the following compound: 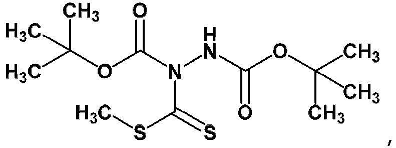 the reaction temperature is preferably 0°C or higher, more preferably 10°C or higher throughout the reaction from the start to the end. The reaction temperature is preferably 50°C or lower, and more preferably 40°C or lower throughout the reaction from the start to the end. Therefore, the reaction temperature is preferably 0 to 50°C, and more preferably 10 to 40°C throughout the reaction from the start to the end.

### Examples

The features of the present disclosure are described in more detail below with reference to Examples. However, the present disclosure is not limited to the embodiments of these Examples.

### Examples 1 to 5

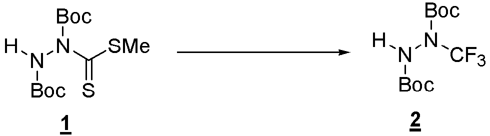

In the formula, Boc represents a tert-butyloxycarbonyl group, and Me represents a methyl group.

### Example 1

Toluene (1.3 g) was added to a reactor, followed by the addition of raw material compound 1 (1.3 g, 4.03 mmol). While stirring, IF₅-Et₃N-3HF (1.54 g, 4.03 mmol) was added thereto dropwise and reacted at room temperature for 16 hours (during the dropwise addition and reaction, the reaction liquid generates heat; therefore, the external temperature was adjusted to maintain the internal temperature at 10°C to 40°C). After terminating the reaction with an aqueous potassium hydroxide solution, the yield of target compound 2 was confirmed to be 70% by F-NMR.

### Example 2

The reaction was carried out in the same manner as in Example 1, except that the solvent was changed from toluene to dichloromethane, thereby obtaining target compound 2 in a yield of 66%.

### Example 3

The reaction was carried out in the same manner as in Example 1, except that the solvent was changed from toluene to n-heptane, thereby obtaining target compound 2 in a yield of 59%.

### Example 4

The reaction was carried out in the same manner as in Example 1, except that the solvent was changed from toluene to ethyl acetate, thereby obtaining target compound 2 in a yield of 48%.

### Example 5

The reaction was carried out in the same manner as in Example 1, except that the solvent was changed from toluene to acetonitrile, thereby obtaining target compound 2 in a yield of 53%.

### Comparative Example 1

The reaction was carried out in the same manner as in Example 1, except that IF₅-Et₃N-3HF was added while stirring the reaction liquid at room temperature (internal temperature: 27°C), without adjusting the external temperature. It was confirmed that the internal temperature increased to 95°C. Consequently, target compound 2 was obtained in a yield of 15%.

### Example 6

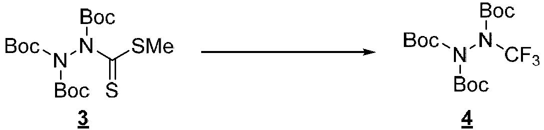

In the formula, Boc represents a tert-butyloxycarbonyl group, and Me represents a methyl group.

Toluene (1.7 g) was added to a reactor, followed by the addition of raw material compound 3 (1.7 g, 4.03 mmol). While stirring, IF₅-Et₃N-3HF (1.85 g, 4.84 mmol) was added thereto dropwise and reacted at room temperature for 19 hours (during the dropwise addition and reaction, the reaction liquid generates heat; therefore, the external temperature was adjusted to maintain the internal temperature at 10°C to 40°C). After terminating the reaction with an aqueous potassium hydroxide solution, the yield of target compound 4 was confirmed to be 27% by F-NMR.

### Example 7

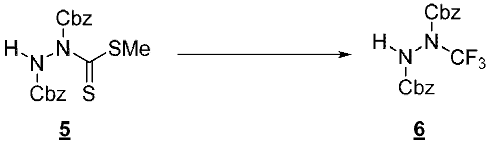

In the formula, Cbz represents a benzyloxycarbonyl group, and Me represents a methyl group.

Dichloromethane (290 g) was added to a reactor, followed by the addition of raw material compound 5 (1.82 g, 4.03 mmol). While stirring, IF₅-Et₃N-3HF (3.87 g, 10.1 mmol) was added thereto dropwise and reacted at room temperature for 14.5 hours (during the dropwise addition and reaction, the reaction liquid generates heat; therefore, the external temperature was adjusted to maintain the internal temperature at 10°C to 40°C). After terminating the reaction with an aqueous potassium hydroxide solution, the yield of target compound 6 was confirmed to be 23% by F-NMR.

### Example 10 to 11

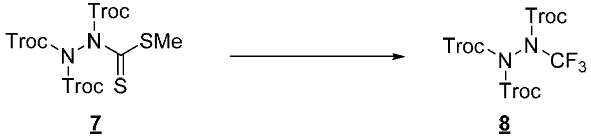

In the formula, Troc represents a (2,2,2-trichloroethoxy)carbonyl group, and Me represents a methyl group.

### Example 10

Dichloromethane (7.3 g) was added to a reactor, followed by the addition of raw material compound 7 (2.61 g, 4.03 mmol). While stirring, IF₅-Et₃N-3HF (6.95 g, 18.1 mmol) was added thereto dropwise and reacted at room temperature for 25 hours (during the dropwise addition and reaction, the reaction liquid generates heat; therefore, the external temperature was adjusted to maintain the internal temperature at 10°C to 40°C). After terminating the reaction with an aqueous potassium hydroxide solution, the yield of target compound 8 was confirmed to be 25% by F-NMR.

### Example 11

The reaction was carried out in the same manner as in Example 10, except that the reaction temperature was maintained at an internal temperature of 50°C to 60°C instead of 10°C to 40°C, and that the solvent was changed from dichloromethane to 1,2-dichloroethane, thereby obtaining target compound 8 in a yield of 22%.

### Example 12

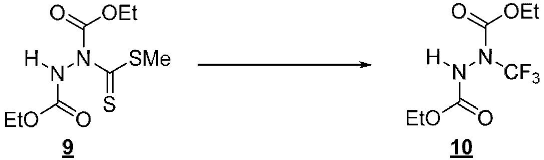

In the formula, Et represents an ethyl group, and Me represents a methyl group.

Dichloromethane (200 g) was added to a reactor, followed by the addition of raw material compound 9 (1.32 g, 4.03 mmol). While stirring, IF₅-Et₃N-3HF (2.78 g, 7.25 mmol) was added thereto dropwise and reacted at room temperature for 18.5 hours (during the dropwise addition and reaction, the reaction liquid generates heat; therefore, the external temperature was adjusted to maintain the internal temperature at 10°C to 40°C). After terminating the reaction with an aqueous potassium hydroxide solution, the yield of target compound 10 was confirmed to be 23% by F-NMR.

### Example 13

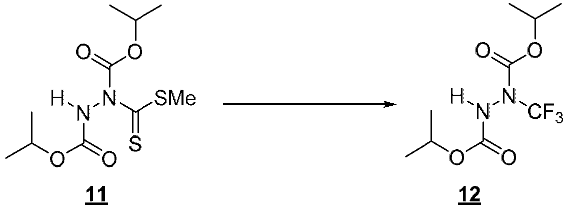

In the formula, Me represents a methyl group.

Dichloromethane (200 g) was added to a reactor, followed by the addition of raw material compound 11 (1.44 g, 4.03 mmol). While stirring, IF₅-Et₃N-3HF (3.70 g, 9.67 mmol) was added thereto dropwise and reacted at room temperature for 18.5 hours (during the dropwise addition and reaction, the reaction liquid generates heat; therefore, the external temperature was adjusted to maintain the internal temperature at 10°C to 40°C). After terminating the reaction with an aqueous potassium hydroxide solution, the yield of target compound 12 was confirmed to be 22% by F-NMR.

## Claims

1. A method for producing a compound represented by formula (1): 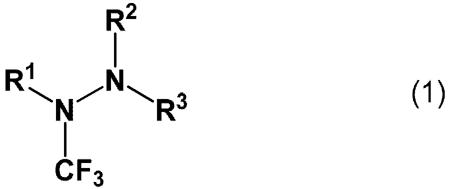 wherein
R¹, R², and R³ are the same or different,
R¹ represents -COR, and
R² and R³ each represent a hydrogen atom or -COR,
wherein Rs are the same or different and each represents a C₁₋C₁₂ alkoxy group optionally substituted with one or more substituents or a C₆-C₁₂ aryloxy group optionally substituted with one or more substituents;
the method comprising step A of reacting a compound represented by formula (2): 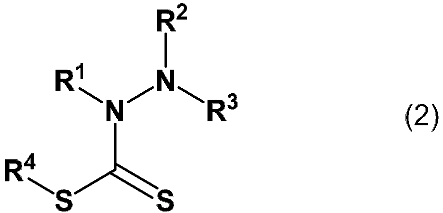 wherein
R¹, R², and R³ are as defined above, and
R⁴ represents a C₁₋C₁₂ alkyl group or a C₆-C₁₂ aryl group,
with a fluorinating agent containing IF₅, an amine, and HF at 80°C or lower throughout the reaction from the start to the end.

2. The production method according to claim 1, wherein in formulas (1) and (2),
R¹ represents a tert-butyloxycarbonyl group, a benzyloxycarbonyl group, a (2,2,2-trichloroethoxy)carbonyl group, an ethoxycarbonyl group, or an isopropoxycarbonyl group, and
R² and R³ are the same or different and each represents a hydrogen atom, a tert-butyloxycarbonyl group, a benzyloxycarbonyl group, a (2,2,2-trichloroethoxy)carbonyl group, an ethoxycarbonyl group, or an isopropoxycarbonyl group.

3. The production method according to claim 1 or 2,
wherein the compound represented by formula (2) is

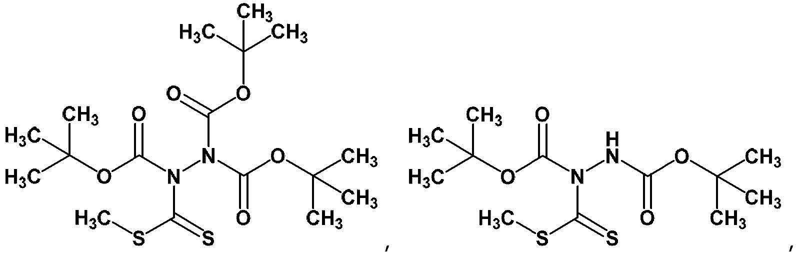

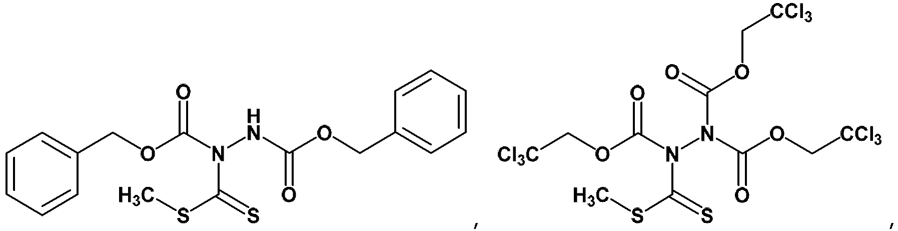

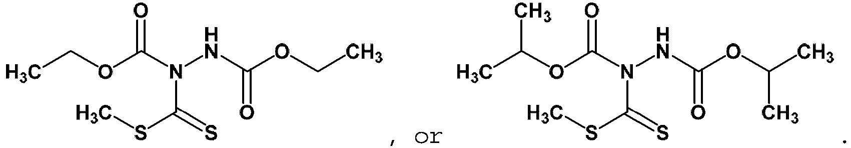

4. The production method according to claim 3, wherein the compound represented by formula (2) is

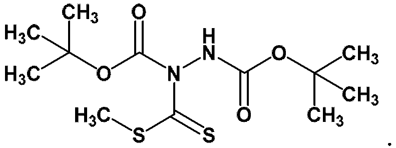

5. The production method according to any one of claims 1 to 4, wherein the compound represented by formula (1) is

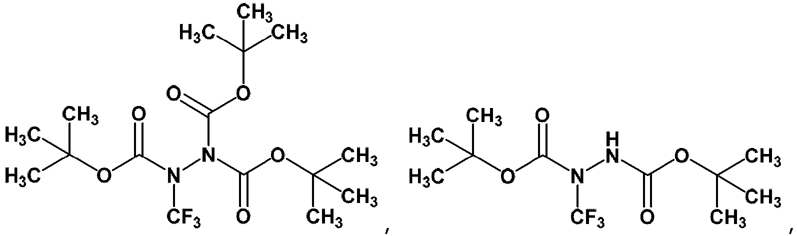

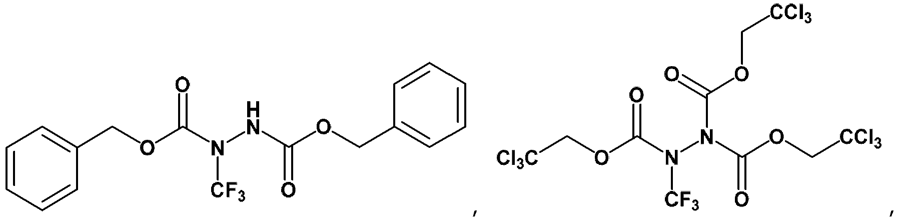

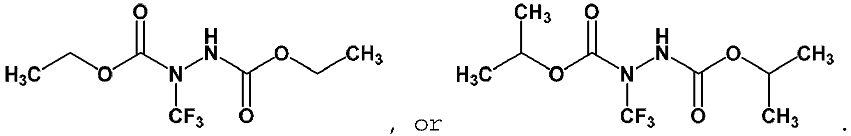

6. The production method according to claim 5, wherein the compound represented by formula (1) is

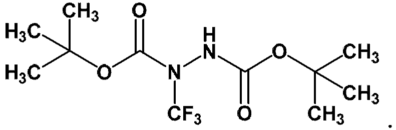

7. The production method according to any one of claims 1 to 6, wherein step A is carried out at a reaction temperature of 10 to 40°C.

8. The production method according to any one of claims 1 to 7, wherein step A is carried out in the presence of a solvent.

9. The production method according to claim 8, wherein the solvent is at least one member selected from the group consisting of (cyclo)alkanes, chlorine-containing organic solvents, aromatic solvents, nitrile-containing organic solvents, fluorine-containing organic solvents, and ester-containing organic solvents.
